# EUROPÄISCHE PATENTSCHRIFT

(11) **EP 3 174 509 B1**
(45) Veröffentlichungstag und Bekanntmachung des Hinweises auf die Patenterteilung: **13.06.2018**
(21) Anmeldenummer: 15744570.1
(22) Anmeldetag: 30.07.2015
(51) Int. Cl.: A61F 13/15, A61F 13/56

(54) **VERFAHREN ZUM HERSTELLEN VON INKONTINENZWEGWERFWINDELN**
METHOD FOR PRODUCING DISPOSABLE INCONTINENCE DIAPERS
PROCÉDÉ DE FABRICATION DE COUCHES JETABLES POUR INCONTINENCE

(30) Priorität: 01.08.2014 DE 102014110941
(43) Veröffentlichungstag der Anmeldung: 07.06.2017
(73) Patentinhaber: Paul Hartmann AG, 89522 Heidenheim (DE)
(72) Erfinder: KNECHT, Theresia, 73433 Aalen (DE)
(86) Internationale Anmeldenummer: PCT/EP2015/067541
(87) Internationale Veröffentlichungsnummer: WO 2016/016384

(56) Entgegenhaltungen:
- EP-A1- 1 941 853
- EP-A2- 2 581 068
- WO-A1-2011/101772
- WO-A1-2014/076626

## Beschreibung

Die Erfindung betrifft ein Verfahren zum Herstellen einer Vielzahl von einen Windelhauptteil und daran angefügte vordere und hintere Windelseitenteile aufweisenden Inkontinenzwegwerfwindeln des offenen Typs für Erwachsene. Derartige Inkontinenzwegwerfwindeln sind bekannt und weisen einen Hauptteil, bestehend aus einem Vorderbereich, einem Rückenbereich und einem in Längsrichtung dazwischen liegenden zwischen den Beinen eines Benutzers zu liegen kommenden Schrittbereich auf, wobei der Hauptteil meist bereits einen Absorptionskern umfasst, und mit an den Rückenbereich und/oder an den Vorderbereich beidseits angefügten, voneinander separaten Windelseitenteilen, welche sich in Querrichtung über seitliche Längsränder des Hauptteils hinaus erstrecken und den Vorderbereich und den Rückenbereich im angelegten Zustand des Artikels miteinander verbinden.

Die Seitenteile werden vorzugsweise im Cut&Place-Verfahren (Slip-Cut) direkt, beidseitig an den Windelhauptteil, also das Chassis des Hygieneartikels angefügt. Diese Fertigungstechnologie erlaubt es, die Seitenteile aus einem anderen Material zu fertigen als den Windelhauptteil des Hygieneartikels. Beispielsweise können die Windelseitenteile luftdurchlässig ausgeführt werden, wohingegen der Windelhauptteil im Wesentlichen feuchtigkeitsundurchlässig ausgebildet werden kann.

Die aus der Fertigungssicht effizienteste und einfachste sowie kostengünstigste Form der Windelseitenteile ist die rechteckige Form. Sie erlaubt bei der Herstellung den Transport der die Windelseitenteile bildenden Materialien in Form einer endlosen Flachmaterialbahn, von der dann die Windelseitenteile quer zur Maschinenrichtung abgetrennt werden. Ein Schnittabfall ist hier praktisch nicht gegeben.

Es hat sich jedoch gezeigt, dass insbesondere bei der Ausbildung der Windelseitenteile in der ansonsten vorteilhaften Rechteckform beim Anlegen und Tragen des Hygieneartikels mitunter das Problem besteht, dass die angefügten Windelseitenteile im Bereich der seitlichen Längsränder des Windelhauptteils einreißen können. Die Anwender sind nämlich geneigt, beim Anlegen des Hygieneartikels einen zur Quer- und Längsrichtung des Hygieneartikels schrägen Zug auf die im Falle von Inkontinenzartikeln für Erwachsene extrem weit in Quer-und Längsrichtung ausladenden Windelseitenteile auszuüben. Es kann solchenfalls vorkommen, dass Windelseitenteile entlang der seitlichen Längsränder des Windelhauptteils einreißen, wobei der Riss ausgehend von dem dem Schrittbereich zugewandten Querrand des Windelseitenteils ausgeht. Die DE 102010026643 schlägt daher vor, die hinteren Windelseitenteile mit einer Konturierung zu versehen. Die DE102010049171 zeigt darüber hinaus bereits ein Verfahren zur Herstellung einer Inkontinenzwegwerfwindel mit konturierten Windelseitenteilen. So zeigt die DE 102010049171 auch bereits die weitestgehend materialverlustfreie Erzeugung von Windelseitenteilen aus einer endlosen Materialbahn von der schräg zur Maschinenrichtung der Materialbahn doppelnutzige Materialbahnabschnitte in alternierender Abfolge abgetrennt werden. Auch WO2008141834 und DE102008046358A1 offenbaren Verfahren zur Herstellung von Inkontinenzwegwerfwindeln der eingangs beschriebenen Art. Es hat sich jedoch gezeigt, dass das präzise Führen der Materialbahn in schnell laufenden Windelherstellungsmaschinen mit Schwierigkeiten versehen ist. Beim Schneiden und Abtrennen der Materialbahnabschnitte von der Materialbahn muss die Materialbahn unter Zug gehalten werden, was angesichts der erforderlichen Breite der Materialbahn und der oft geringen Flächengewichte der verwendeten Materialien problematisch ist. Der vorliegenden Erfindung liegt daher die Aufgabe zu Grunde, ein verbessertes Verfahren zum Herstellen von derartigen oder ähnlichen Inkontinenzwegwerfwindeln zu schaffen, das kostengünstig und prozesstechnisch vorteilhaft realisierbar ist.

Diese Aufgabe wird erfindungsgemäß gelöst durch ein Verfahren mit den folgenden Verfahrensschritten: Fördern einer Windelseitenteile bildenden Materialbahn in einer Maschinenrichtung, wobei die Materialbahn einen ersten und einen zweiten Materialbahnrand aufweist; Ausführen von ersten Schnitten durch die Materialbahn hindurch und schräg zu der Maschinenrichtung zur Bildung von der Materialbahn abzutrennender doppelnutziger Materialbahnabschnitte, welche entlang der Materialbahn in der Maschinenrichtung alternierend angeordnet sind, derart, dass in der Maschinenrichtung direkt aufeinanderfolgende Materialbahnabschnitte zueinander in der Ebene um 180° gedreht angeordnet sind, wobei erste Trennlinien der ersten Schnitte sich nicht bis zu den ersten und zweiten Materialbahnrändern der Materialbahn erstrecken (so dass die Materialbahn zunächst endlos bleibt); Faltung der Materialbahn wenigstens um zwei in Maschinenrichtung verlaufende Falzlinien zu einer Faltanordnung , wobei zumindest eine der Falzlinien die ersten Trennlinien kreuzt; Abtrennen von doppelnutzigen Materialbahnabschnitten von der Materialbahn quer oder schräg zu der Maschinenrichtung, derart, dass die ersten Trennlinien zumindest bereichsweise die schräg zur Längsachse der Inkontinenzwegwerfwindel verlaufenden oder konturierten Beinöffnungsbereiche der Inkontinenzwegwerfwindel bilden können.

Dadurch, dass die ersten Trennlinien sich nicht bis zu den Materialbahnrändern der ersten Materialbahn erstrecken, lässt sich die Materialbahn bis zum endgültigen Abtrennen der doppelnutzigen Materialbahnabschnitte unter Spannung halten, was der Präzision der Fertigung und der Ermöglichung hoher Maschinengeschwindigkeiten zugute kommt. Vorteilhafterweise erstrecken sich die ersten Trennlinien über 50-95 % der Breite der Materialbahn, insbesondere über 60-90 % der Materialbahn, insbesondere über 65-85% der Materialbahn.

Durch das Einfalten der Bahn wird die Breite der Bahn reduziert. Auch das erleichtert die nachfolgenden Verfahrensschritte. Die in der Maschinenrichtung alternierende Anordnung der von der Bahn abzutrennenden doppelnutzigen Materialbahnabschnitte erlaubt eine weitestgehend materialverlustfreie Bereitstellung der Windelseitenteile. Die alternierende Anordnung der von der Bahn abzutrennenden Materialbahnabschnitte bedeutet im Zusammenhang mit der vorliegenden Erfindung, dass ein jeweiliger Materialbahnabschnitt in der Ebene um 180° gedreht angeordnet ist gegenüber einem unmittelbar in der Maschinenrichtung nachfolgenden Materialbahnabschnitt. Somit bildet der erste Materialbahnrand einen inneren Rand eines ersten Materialbahnabschnittes und der zweite Materialbahnrand einen äußeren Rand des ersten Materialbahnabschnittes und der zweite Materialbahnrand einen inneren Rand eines zweiten Materialbahnabschnittes und der erste Materialbahnrand einen äußeren Rand des zweiten Materialbahnabschnittes, wobei der zweite Materialbahnabschnitt in der Maschinenrichtung unmittelbar auf den ersten Materialbahnabschnitt folgt. Hierbei ist ein innerer Rand eines Materialabschnitts derjenige Rand, welcher im weiteren Verlauf der Herstellung der Inkontinenzwegwerfwindel an den Seitenrand einer Hauptteilbahn angefügt wird.

Die ersten Trennlinien können vorteilhaft lediglich durch sich durch die Materialbahn bereichsweise in z-Richtung hindurcherstreckende Schnitte ausgeführt sein. Vorzugsweise bilden die Trennlinien hingegen Materialaussparungen, derart, dass jeweils eine langgestreckte Öffnung insbesondere in ovaler Form oder einem Oval ähnlicher Form ausgebildet wird. Solchenfalls ist eine jeweilige Öffnung in der Ebene der Materialbahn vollumfänglich von Material der Materialbahn umschlossen. Durch das Ausbilden von Materialaussparungen, vorzugsweise vermittels entsprechender Messerwalzen, kann den späteren Windelseitenteilen die gewünschte Kontur gegeben werden. Des Weiteren erleichtert das Ausbilden von Öffnungen das endgültige Abtrennen der Materialbahnabschnitte von der Materialbahn.

Vorteilhaft sind in der Maschinenrichtung aufeinanderfolgende erste Trennlinien, insbesondere Öffnungen der Materialbahn jeweils symmetrisch in Bezug auf eine Spiegelachse quer zur Maschinenrichtung ausgerichtet. Vorzugsweise entspricht diese Spiegelachse der Lage der Trennlinie, an welcher in einer nachgelagerten Verfahrensstufe die Inkontinenzwindeln vereinzelt werden, nachdem die doppelnutzigen Materialabschnitte wie weiter unten ausgeführt an einer Hauptteilbahn zuvor angebracht wurden.

Die Breite der noch ungefalteten Materialbahn entspricht im Wesentlichen der Quererstreckung der späteren Windelseitenteile im entfalteten Zustand und beträgt vorzugsweise 10-50 cm, insbesondere 12-45 cm, weiter insbesondere 15-35 cm.

Die Breite der gefalteten Materialbahn beträgt 5-30 cm, insbesondere 6-25 cm.

Es hat sich als vorteilhaft erwiesen, dass die Faltanordnung zumindest eine Z-Falte umfasst. Nach weiteren vorteilhaften Varianten wird die Materialbahn um wenigstens drei, insbesondere um wenigstens vier in Maschinenrichtung verlaufenden Falzlinien gefaltet. Vorzugsweise kreuzen wenigstens zwei, insbesondere wenigstens drei, weiter insbesondere wenigstens vier der Falzlinien die ersten Trennlinien.

Es hat sich als weiter vorteilhaft erwiesen, wenn die in Maschinenrichtung verlaufenden Falzlinien beidseits und insbesondere symmetrisch zu einer Mittellängsachse der Materialbahn angeordnet sind. Insbesondere vorteilhaft ist, wenn die Faltanordnung in Form einer Omega-Faltung ausgebildet ist.

Nach einer alternativen Verfahrensführung können die Falzlinien asymmetrisch zu der Mittellängsachse der Materialbahn angeordnet sein. Insbesondere kann die Faltanordnung zwei Z-Falten umfassen oder daraus bestehen, wobei die eine Z-Falte vorzugsweise in die andere Z-Falte eingeschoben ist, also Teilabschnitte jeweiliger Z-Falten aufeinander zu liegen kommen.

Insbesondere erstreckt sich die Faltanordnung in der Breite der Materialbahn nicht über den ersten und zweiten Materialbahnrand der Materialbahn hinaus. Mithin bilden der erste und zweite Materialbahnrand der noch ungefalteten Materialbahn auch den ersten und zweiten Materialbahnrand der gefalteten Materialbahn.

Es ist weiter vorteilhaft wenn bei der Abtrennung der doppelnutzigen Materialbahnabschnitte von der Materialbahn die Abtrennung ausgehend von einem jeweiligen Materialbahnrand der Materialbahn bis zu der ersten Trennlinie erfolgt. Die erste Trennlinie bildet mithin zumindest bereichsweise die Kontur der späteren Beinöffnungsbereiche der Windelseitenteile. Die Abtrennung kann durch an sich beliebige Trennverfahren insbesondere Schneiden, Stanzen oder Abreißen erfolgen. Auch könnten nach einer weiteren Variante Schwächungslinien wie Perforationen oder Ähnliches eingebracht werden, die - vorzugsweise in einer nachfolgenden Verfahrensstufe - die Abtrennung der Materialbahnabschnitte insbesondere durch Ausübung von Zug- oder Druckkräften ermöglichen.

Die Materialbahnabschnitte sind erfindungsgemäß doppelnutzig ausgeführt. Das bedeutet, dass ein einziger doppelnutziger Materialbahnabschnitt jeweils zwei Windelseitenteile bildet.

Hierbei kann ein doppelnutziger Materialbahnabschnitt zwei hintere Windelseitenteile oder zwei vordere Windelseitenteile oder ein hinteres und ein vorderes Windelseitenteil bilden. Nach einer vorteilhaften Weiterbildung der Erfindung können die doppelnutzigen Materialbahnabschnitte nach der Abtrennung von der Materialbahn um wenigstens je eine weitere in Maschinenrichtung verlaufende Falzlinie gefaltet werden.

Im weiteren Verlauf der Herstellung der Windel kann es vorteilhaft sein, die doppelnutzigen Materialbahnabschnitte einer in der Maschinenrichtung geförderten Hauptteilbahn zuzuführen und die Materialbahnabschnitte zur Bildung von späteren Windelseitenteilen an der Hauptteilbahn anzufügen. Dies kann vorteilhaft mit dem Fachmann an sich bekannten Vorrichtungen, wie so genannten Cut&Place (Slip-Cut) - Applikatoren erfolgen.

Die Hauptteilbahn kann vorteilhaft einen Verbund aus einem Backsheet, einem Topsheet und einen dazwischen angeordneten Saugkörper aufweisen. Die Hauptteilbahn kann nach einer Variante hingegen aus lediglich einer oder zweier dieser Komponenten bestehen. Des Weiteren kann die Hauptteilbahn vorzugsweise eine so genannte Cuff-Komponente aufweisen oder daraus bestehen. Als Cuff-Komponenten werden üblicherweise in Gebrauch flüssigkeitsabweisende oder hydrophobe Flachmaterialien, insbesondere auf Vliesstoffbasis bezeichnet, welche eine sich im wesentlichen über die gesamte Länge zumindest des Schrittbereiches der Windel erstreckende seitliche Auslaufschutzbarriere bilden und vorzugsweise Teil der körperzugewandten Oberseite der Windel sind. Solchenfalls würde die Cuff-Komponente mit daran angefügten spätere Windelseitenteile bildenden Materialbahnabschnitten in einer nachfolgenden Verfahrensstufe mit weiteren, den Windelhauptteil bildenden Komponenten verbunden werden.

Vorteilhafterweise wird die Materialbahn in der Maschinenrichtung mit einer ersten Geschwindigkeit v1 gefördert und die Hauptteilbahn in der Maschinenrichtung mit einer zweiten Geschwindigkeit v2 gefördert, wobei gilt v1 kleiner v2. Solchenfalls werden die Materialbahnabschnitte vorteilhafterweise nach der Abtrennung von der Materialbahn im Zuge der Applikation auf die Windelhauptteilbahn beschleunigt, insbesondere auf eine Geschwindigkeit v3, die im Wesentlichen v2 entspricht.

Vorteilhaft erfolgt nach dem Anfügen der doppelnutzigen Materialabschnitte an die Hauptteilbahn an einer Windelvereinzelungsstation die Vereinzelung der zuvor noch endlosen Hauptteilbahn mit daran angefügten doppelnutzigen Materialabschnitten quer zur Maschinenrichtung, wobei die doppelnutzigen Materialbahnabschnitte erst gleichzeitig mit dem Vereinzelungsschnitt in Windelseitenteile einer jeweils vereinzelten Windel getrennt werden.

Nach einer ersten Variante der Erfindung werden von der Materialbahn doppelnutzige Materialbahnabschnitte abgetrennt, die hintere Windelseitenteile bilden. Solchenfalls ist es vorteilhaft, dass vor dem Abtrennen der Materialbahnabschnitte von der Materialbahn Verschlussmittel auf die Materialbahn aufgebracht und dort festgelegt werden. Die Verschlussmittel werden häufig auch als Tapes bezeichnet, welche die klebende oder mechanische Verschlusselemente aufweisen.

Nach einer Variante der Erfindung werden von der Materialbahn doppelnutzige Materialbahnabschnitte abgetrennt, die vordere Windelseitenteile bilden.

Nach einer weiteren Variante der Erfindung werden von einer ersten Materialbahn doppelnutzige Materialbahnabschnitte abgetrennt, die hintere Windelseitenteile bilden und von einer zweiten Materialbahn werden doppelnutzige Materialbahnabschnitte abgetrennt, die vordere Windelseitenteile bilden.

Nach einer weiteren Ausführungsform des erfindungsgemäßen Verfahrens werden von einer einzigen Materialbahn doppelnutzige Materialbahnabschnitte abgetrennt, die sowohl vordere als auch hintere Windelseitenteile bilden.

Es hat sich als vorteilhaft erwiesen, die Windeln derart zu fertigen, dass bei in der Maschinenrichtung aufeinanderfolgend geförderten Windeln der Rückenbereich der einen Windel an den Rückenbereich der anderen Windel anschließt und der Vorderbereich der einen Windel an den Vorderbereich der anderen Windel anschließt. Nach einer alternativen Verfahrensführung werden die Windeln derart gefertigt, dass bei in der Maschinenrichtung aufeinanderfolgender geförderten Windeln der Rückenbereich der einen Windel an den Vorderbereich der anderen Windel anschließt.

Es hat sich weiter als vorteilhaft erwiesen, die vorderen und/oder hinteren Windelseitenteile aus einem Vliesstoffmaterial zu bilden, welches zumindest eine Rezepturkomponente auf Basis eines thermoplastischen Polymers enthält. Die Vliesstoffe können Fasern aus PE, PP, PET, Rayon, Cellulose, PA und Mischungen dieser Fasern enthalten. Auch Bi- oder Multikomponentenfasern sind denkbar und vorteilhaft. Vorteilhaft sind insbesondere Kardenvliese, Spinnvliese, wasserstrahlvernadelte Vliese, SM-Vliese, SMS-Vliese, SMMS-Vliese oder auch Laminate aus einer oder mehreren dieser Vliesarten, wobei S für Spunbond- und M für Meltblown-Vliesschichten steht. Besonders bevorzugt sind Spinnvliese, da diese eine hohe Festigkeit in Längs- und Querrichtung aufweisen und somit den auf sie durch das Eingreifen von gegebenenfalls vorhandenen mechanischen Verschlusshilfen einwirkenden Scherkräften besonders gut standhalten können.

Vorteilhaft unterscheiden sich die hinteren Windelseitenteile von den vorderen Windelseitenteilen bezüglich mindestens einer, insbesondere mindestens zweier, weiter insbesondere mindestens dreier und weiter insbesondere mindestens vierer ihrer Primäreigenschaften ausgewählt aus der Gruppe Art des Materials, Flächengewicht, Atmungsaktivität, Dichte, Dehnbarkeit, Verschlusskraft, Flächenerstreckung, Dicke, Farbe. Art des Materials: Es erweist sich als vorteilhaft, wenn die vorderen Windelseitenteile aus einem weicheren, hautfreundlicheren Vliesmaterial gebildet sind als die hinteren Windelseitenteile, da die vorderen Windelseitenteile beim Anlegen der Windel an den Körper bestimmungsgemäß innen zu liegen kommen. Weiterhin kann es vorteilhaft sein, die hinteren Windelseitenteile aus einem zugfesteren und/oder durchreißfesteren Material zu bilden, da die Verschlussmittel an den hinteren Windelseitenteilen angebracht sind und beim Anlegen der Windel starke Zugkräfte über die Verschlussmittel auf die Windelseitenteile einwirken. Bevorzugte Differenzierungen hinsichtlich der Art des Materials lassen sich durch die verwendete Faserart oder Faserstärke, das Vliesbildungsverfahren oder Laminatbildungen realisieren.

Flächengewicht: Die zuvor genannten Anforderungen können vorzugsweise zumindest anteilig über eine Differenzierung des Flächengewichtes, gemessen in g/m2, erreicht werden. Vorzugsweise unterscheidet sich das Flächengewicht der vorderen Windelseitenteile um mindestens 10%, insbesondere um mindestens 20% und weiter insbesondere um mindestens 30% von dem der hinteren Windelseitenteile. Weiter vorzugsweise unterscheidet sich das Flächengewicht der vorderen und/oder hinteren Windelseitenteile um mindestens 10%, insbesondere um mindestens 20% und weiter insbesondere um mindestens 30% von dem Flächengewicht des Hauptteils der Windelhülle. Das Flächengewicht der vorderen und/oder der hinteren Windelseitenteile und/oder des Hauptteils der Windelhülle beträgt vorzugsweise 8-60 g/m², insbesondere 10-45 g/m², weiter insbesondere 12-40 g/m² und weiter insbesondere 15-35 g/m².

Atmungsaktivität: Da das subjektive Empfinden der Beeinträchtigung des Tragekomforts von Zielgruppe zu Zielgruppe (beispielsweise bettlägerige Patienten vs. mobile Patienten) verschieden ist, kann es vorteilhaft sein, die Atmungsaktivität entweder der vorderen oder der hinteren Windelseitenteile höher auszubilden. Vorzugsweise unterscheidet sich die Atmungsaktivität gemessen als Wasserdampfdurchlässigkeit (WVTR nach DIN 53 122-1 Ausgabe: 2001-08) der vorderen Windelseitenteile von der der hinteren Windelseitenteile um mindestens 5%, insbesondere um mindestens 10% und weiter insbesondere um mindestens 20%. Vorzugsweise beträgt dabei die Atmungsaktivität der vorderen und/oder hinteren Windelseitenteile mindestens 1000 g/m²/24h, insbesondere mindestens 1500 g/m²/24h, weiter vorzugsweise mindestens 2000 g/m²/24h.

Dichte und Dicke: Die subjektiv empfundene Weichheit des Windelseitenteilmaterials und damit eine wesentliche Komponente des Tragekomforts kann vorteilhaft auch über eine Differenzierung der Dichte und/oder Dicke des Materials gesteuert werden. Vorzugsweise unterscheidet sich die Dicke gemessen in mm, ermittelt bei einem Prüfdruck von 0,5 kPa, und/oder die Dichte gemessen in g/cm³, ermittelt aus den Größen Flächengewicht und Dicke des Materials, der vorderen Windelseitenteile um mindestens 15%, insbesondere um mindestens 20% und weiter insbesondere um mindestens 25% von der Dichte und/oder der Dicke der hinteren Windelseitenteile.

Dehnbarkeit: Unter Dehnung wird vorliegend das Verhältnis zwischen einer Längenzunahme eines Windelseitenteils der Inkontinenzwegwerfwindel infolge einer Krafteinwirkung und der ursprünglichen Länge verstanden. In Gebrauch derartiger Inkontinenzwegwerfwindeln wirken auf die Windelseitenteile insbesondere Kräfte in Umfangs- also Windelquerrichtung. Mit der Eigenschaft Dehnbarkeit ist somit das Ausmaß der Dehnung des Windelseitenteils bei Einwirken einer Kraft in Windelquerrichtung gemeint. Das heißt, je höher das Ausmaß der Dehnung, desto höher ist die Dehnbarkeit. Vorzugsweise weist ein hinteres Windelseitenteil bei in Gebrauch der Windel üblicher Krafteinwirkung eine größere Dehnbarkeit auf als ein vorderes Windelseitenteil. Insbesondere weist nach der in DE102005048868A1 beschriebenen Prüfmethode ein hinteres Windelseitenteil bei einer Krafteinwirkung von 45 N eine höhere Dehnung auf als ein vorderes Windelseitenteil. Vorzugsweise weist ein hinteres Windelseitenteil bei einer Krafteinwirkung von 45 N eine Dehnung von mindestens 20%, insbesondere mindestens 25% und weiter insbesondere mindestens 30% auf. Ein vorderes Windelseitenteil hingegen weist bei einer Krafteinwirkung von 45 N lediglich eine Dehnung von vorzugsweise höchstens 15%, insbesondere höchstens von 10% und weiter insbesondere von höchstens 8% auf. Vorzugsweise ist zumindest ein hinteres Windelseitenteil zumindest in Querrichtung elastisch dehnbar. Die Dehnbarkeit des Windelseitenteils wird als elastisch bezeichnet, wenn bei kurzzeitiger Einwirkung einer Kraft (2-5 Sekunden) eine Dehnung von mindestens 40% möglich ist und bei Wegnahme dieser Kraft eine Dehnung (bleibende Dehnung) von höchstens 20% verbleibt. In einer vorteilhaften Weiterbildung der Erfindung beträgt die elastische Dehnbarkeit eines hinteren Windelseitenteils in Querrichtung mindestens 40%, insbesondere mindestens 50%. Nach einem weiteren Erfindungsgedanken beträgt das absolute Ausmaß der elastischen Dehnung eines hinteren Windelseitenteils mindestens 3 cm, insbesondere mindestens 5 cm und weiter insbesondere mindestens 7 cm.

Verschlusskraft: Unter Verschlusskraft der Windelseitenteile wird die Haltekraft zwischen den Verschlussmitteln der hinteren Windelseitenteile und der Außenseite der Windelseitenteile verstanden. Vorzugsweise sind die Haltekräfte zwischen den Verschlussmitteln und der Außenseite der hinteren Windelseitenteile hierbei geringer als die Haltekräfte zwischen den Verschlussmitteln und der Außenseite der vorderen Windelseitenteile. Dies führt in vorteilhafter Weise dazu, dass der Benutzer die Verschlussmittel bevorzugt an den vorderen Windelseitenteilen festlegt, was Passform und Tragekomfort der Windel deutlich zugute kommt. Die vorstehend oder nachfolgend erwähnten Haltekräfte werden vorzugsweise als Über-Bauch-Haltekräfte ermittelt. Die Über-Bauch-Haltekräfte sind im Rahmen dieser Erfindung nach der in EP 1915977A1 beschriebenen Prüfmethode zu bestimmen. Die Haltekräfte als Über-Bauch-Haltekräfte ermittelt zwischen den insbesondere mechanischen Verschlusshilfen aufweisenden Verschlussmitteln und der Außenseite der vorderen Windelseitenteile betragen vorzugsweise 40-98N/25mm, insbesondere 45-90N/25mm.

Die Über-Bauch-Haltekräfte zwischen den insbesondere mechanischen Verschlusshilfen aufweisenden Verschlussmitteln und der Außenseite der hinteren Windelseitenteile sind vorzugsweise geringer als die Über-Bauch-Haltekräfte zwischen den Verschlussmitteln und der Außenseite der vorderen Windelseitenteile, sie betragen vorzugsweise aber gleichwohl mindestens 15 N/25mm, insbesondere mindestens 20 N/25mm.

Flächenerstreckung: In Weiterbildung der Erfindung erweist es sich als vorteilhaft, wenn die hinteren Windelseitenteile eine größere, vorzugsweise eine um mindestens 10 %, insbesondere eine um mindestens 15% größere Flächenerstreckung aufweisen als die vorderen Windelseitenteile. Insbesondere kann die Länge der hinteren Windelseitenteile, also deren Erstreckung in Windellängsrichtung mindestens 10 cm, insbesondere mindestens 15 cm, weiter insbesondere mindestens 18 cm und weiter insbesondere mindestens 22 cm betragen. Es erweist sich weiterhin als vorteilhaft, wenn die Länge der hinteren Windelseitenteile mindestens 10 %, insbesondere mindestens 15%, weiter insbesondere mindestens 20% und weiter insbesondere mindestens 22% der Gesamtlänge L1 des Hauptteils beträgt. Vorteilhafterweise beträgt die Gesamtlänge L1 des Hauptteils 50-120 cm, insbesondere 60-110 cm und weiter insbesondere 70-110 cm.

Weiterhin erweist es sich als vorteilhaft, wenn die vorderen Windelseitenteile eine geringere, insbesondere eine um mindestens 5%, weiter insbesondere eine um mindestens 10%, weiter insbesondere eine um mindestens 15% und weiter insbesondere eine um höchstens 50% geringere Längserstreckung aufweisen als die hinteren Windelseitenteile.

Weiterhin erweist es sich als vorteilhaft, wenn die Breite der Windelseitenteile, also die Erstreckung der Windelseitenteile in Querrichtung über den hinteren seitlichen Längsrand des Hauptteils hinaus 10-50 cm, insbesondere 12-45 cm, weiter insbesondere 15-35 cm beträgt. Vorzugsweise weisen die vorderen Windelseitenteile die gleiche Breite auf wie die hinteren Windelseitenteile.

Alle vorstehend beschriebenen Abmessungen der Windelseitenteile werden an eben ausgefalteten Windelseitenteilen ermittelt.

Farbe: Schließlich kann es vorteilhaft sein, die vorderen von den hinteren Windelseitenteilen hinsichtlich der Farbe zu differenzieren. Auch dies kann den Benutzern die Funktion der vorderen Windelseitenteile als bevorzugte Landefläche der Verschlussmittel verdeutlichen. Hinsichtlich der vorstehend beschriebenen vorteilhaften Eigenschaften der vorderen und hinteren Windelseitenteilen ist bevorzugt, dass sich bereits das die Materialbahn bildende Material hinsichtlich der Primäreigenschaften ausgewählt aus der Gruppe Art des Materials, Flächengewicht, Atmungsaktivität, Dichte, Dehnbarkeit, Verschlusskraft, Dicke, Farbe wie vorstehend für die Windelseitenteile beschrieben voneinander unterscheidet, jedenfalls für den Fall, dass von einer ersten Materialbahn doppelnutzige Materialbahnabschnitte abgetrennt werden, die hintere Windelseitenteile bilden und von einer zweiten Materialbahn doppelnutzige Materialbahnabschnitte abgetrennt, die vordere Windelseitenteile bilden.

In den Figuren zeigen:
Figur 1 eine Draufsicht auf eine erfindungsgemäß hergestellte Inkontinenzwegwerfwindel
Figur 2 eine Draufsicht auf eine in der Maschinenrichtung geförderte Materialbahn zur Bildung von hinteren Windelseitenteilen
Figur 3 eine Draufsicht auf eine in der Maschinenrichtung geförderte Materialbahn nach dem Falten der Materialbahn
Figur 4 einen Schnitt durch die Materialbahn der Figur 3 entlang A-A
Figur 5 einen Schnitt durch eine Variante der gefalteten Materialbahn
Figur 6 eine Draufsicht auf eine in der Maschinenrichtung geförderte Materialbahn zur Bildung von vorderen Windelseitenteilen
Figur 7 schematisch das Abtrennen von doppelnutzigen Materialbahnabschnitten von einer Materialbahn zur Bildung von vorderen Windelseitenteilen
Figur 8 schematisch das Anfügen der doppelnutzigen Materialbahnabschnitte an eine Hauptteilbahn
Figur 9 das Vereinzeln der Hauptteilbahn mit daran angefügten doppelnutzigen Materialbahnabschnitten zur Bildung von Inkontinenzwegwerfwindeln

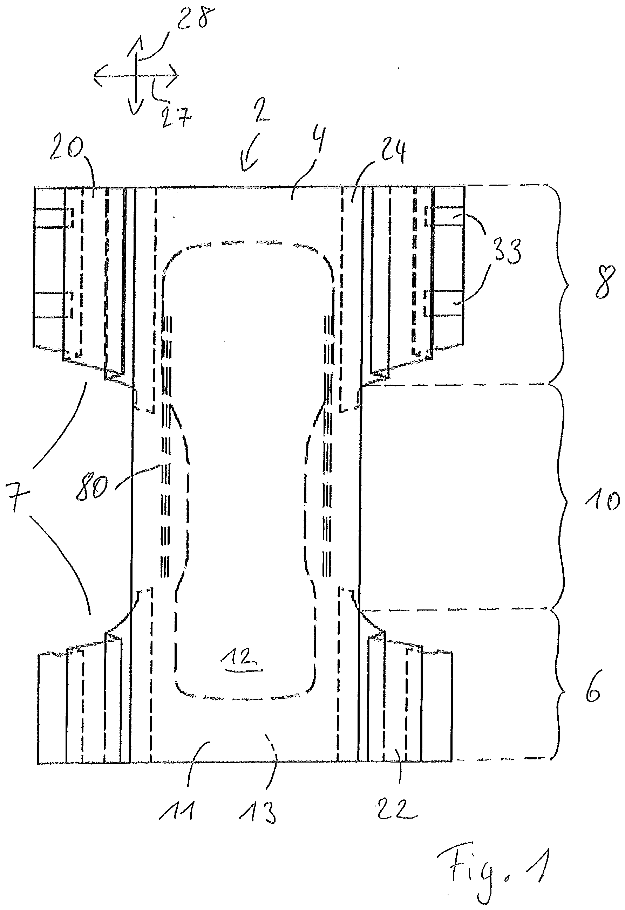

Figur 1 zeigt schematisch, nicht maßstabsgerecht eine Draufsicht auf die Innenseite, also die körperzugewandte Seite einer erfindungsgemäß hergestellten absorbierenden Inkontinenzwegwerfwindel 2 des offenen Typs (nachfolgend auch kurz als "Windel" bezeichnet). Die Windel 2 umfasst einen Hauptteil 4 mit einem Vorderbereich 6, einem Rückenbereich 8 und einem in der Längsrichtung 28 dazwischen liegenden Schrittbereich 10. Außerdem angedeutet ist ein Absorptionskern 12, der üblicherweise zwischen chassisbildenden Materialien des Hauptteils 4, also insbesondere zwischen einem flüssigkeitsdurchlässigen, beispielsweise aus einem Vliesstoffmaterial gebildeten Topsheet 11 und einem im Wesentlichen flüssigkeitsundurchlässigen, beispielsweise aus einem Folienmaterial gebildeten oder ein Folienmaterial aufweisenden Backsheet 13 angeordnet ist. Das Backsheet 13 kann auch aus einem flüssigkeitsundurchlässigem Vliesstoff oder einem Vliesfolienlaminat ausgebildet sein, wobei die Vlieslage dann außen und die Folienlage innen zum Absorptionskern gerichtet zu liegen kommt. Seitlich neben den Längsrändern des Absorptionskerns 12 sind erste elastische Elemente 80 an den Windelhauptteil 4, insbsondere zwischen Topsheet 11 und Backsheet 12 angefügt. Die elastischen Elemente 80 verlaufen im Wesentlichen in der Längsrichtung 28, also mit einer wesentlichen Komponente in Längsrichtung 28.

Die Windel 2 umfasst des Weiteren vordere Windelseitenteile 22 und hintere Windelseitenteile 20, die als vier separate Vliesstoffkomponenten beidseits an den Hauptteil 4 angefügt sind. Die Windelseitenteile 20, 22 weisen jeweils eine Faltanordnung auf. Die Faltanordnung wird in noch näher zu beschreibender Weise im Zuge der Herstellung der Windel eingebracht. Die Windelseitenteile 20, 22 sind in einem Überlappungsbereich 24 mit chassisbildenden Materialien des Hauptteils 4, also beispielsweise mit dem Backsheet 13 oder dem Topsheet 11 unlösbar verbunden. Die Windelseitenteile 20,22 erstrecken sich über die vorderen und hinteren seitlichen Längsränder des Windelhauptteils in Querrichtung 27 hinaus. Windelseitenteile 20, 22 sind dafür gedacht und bestimmt, im angelegten Zustand der Inkontinenzwegwerfwindel 2 miteinander verbunden zu werden, um einen in Umfangsrichtung durchgehenden Hüftbereich der Windel zu bilden. Dabei werden jeweils die auf einer Seite des Windelhauptteils 4 vorgesehenen Windelseitenteile 20, 22 miteinander in Überlappung gebracht. Hierzu sind an den hinteren Windelseitenteilen 20 Verschlussmittel 33, insbesondere mit mechanischen Verschlusshilfen wie Kletthaken, vorgesehen, welche auf der Außenseite der vorderen Windelseitenteile 22 lösbar festlegbar sind. Vorzugsweise sind die Verschlussmittel außerdem auf der Außenseite des Windelhauptteils 4 und weiter vorzugsweise auch auf der Außenseite der hinteren Windelseitenteile 20 lösbar festlegbar, so dass der Inkontinenzartikel sehr variabel an die anatomischen Gegebenheiten des Trägers angepasst werden kann. Sowohl die vorderen Windelseitenteile 22 als auch die hinteren Windelseitenteile 20 sind vorzugsweise aus einem Vliesstoffmaterial, im dargestellten Fall aus einem PP-Spinnvlies gebildet. Das Flächengewicht des Vliesstoffmaterials der vorderen und hinteren Windelseitenteile beträgt 27 g/m². Die Faserstärke der das Vliesstoffmaterial bildenden Fasern beträgt zwei dtex. Wie aus Figur 1 erkennbar ist, weisen die hinteren Windelseitenteile 20 eine größere Flächenerstreckung auf als die vorderen Windelseitenteile 22.

Ein jeweiliger äußerer Rand der Windelseitenteile 20, 22 ist in der Längsrichtung 28 kürzer ausgebildet als ein jeweiliger innerer Rand der Windelseitenteile 20, 22. Mithin sind die Windelseitenteile 20, 22 auf der dem Schrittbereich 10 zugewandten Seite schräg zur Längsrichtung 28 der Windel oder konturiert ausgebildet, um entsprechend schräg oder konturiert ausgebildete Beinöffnungsbereiche 7 zu bilden.

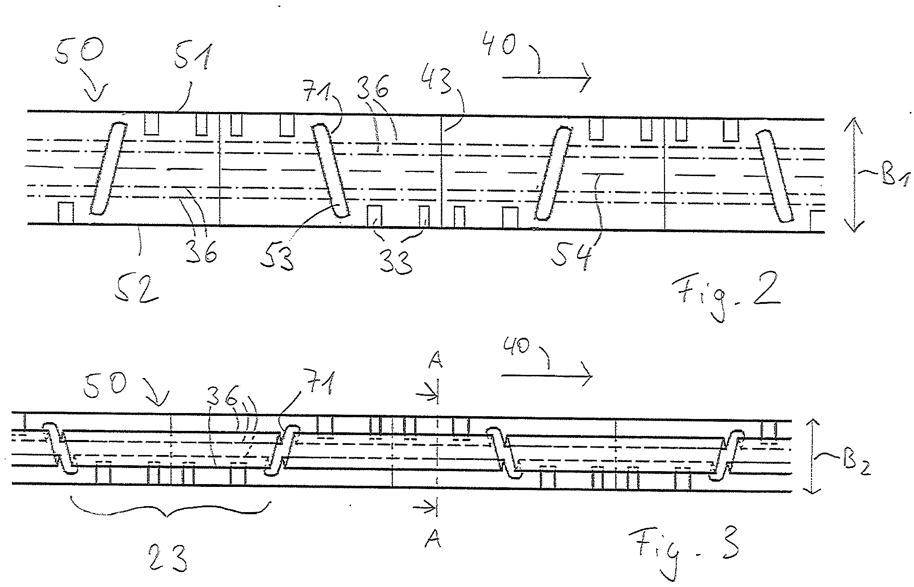

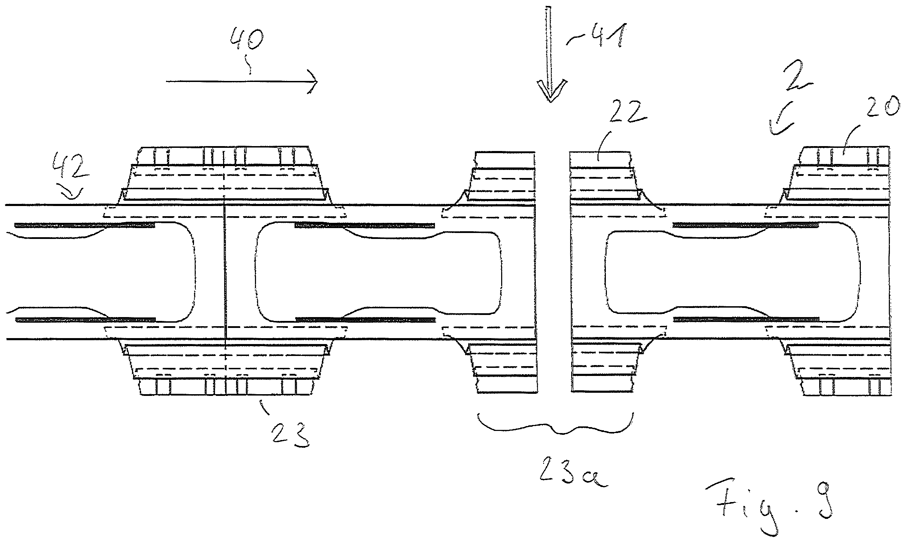

Die Figuren 2-7 zeigen schematisch das Zuführen und Konfigurieren von Materialbahnen 50, 50a, aus denen die vorderen und hinteren Windelseitenteile 20, 22 der Windel 2 gebildet werden. Figur 2 zeigt eine in der Maschinenrichtung 40 geförderte endlose Materialbahn 50. Die Materialbahn 50 weist einen ersten 51 und einen zweiten Materialbahnrand 52 auf. Schräg zu der Maschinenrichtung 40 sind an vorgelagerten Schnittstationen (nicht dargestellt) zur Bildung von im späteren Verlauf abzutrennenden doppelnutzigen Materialbahnabschnitten erste Schnitte ausgeführt, wobei erste Trennlinien 71 der ersten Schnitte sich nicht bis zu den Materialbahn Rändern 51, 52 der Materialbahn 50 erstrecken, so dass die Bahn zunächst endlos bleibt. Die ersten Trennlinien 71 bilden insbesondere Materialaussparungen, derart dass jeweils eine lang gestreckte Öffnung 53 in ovaler Form ausgebildet wird. Eine jeweilige Öffnung 53 ist in der Ebene der Materialbahn 50 vollumfänglich umschlossen. Die Öffnungen 53 weisen hinsichtlich ihrer größten Erstreckung eine Orientierung schräg zu der Maschinenrichtung 40 auf. Hierbei sind aufeinanderfolgende Öffnungen 53 vorzugsweise jeweils symmetrisch in Bezug auf eine Spiegelachse 43 quer zur Maschinenrichtung 40 ausgerichtet. Vorzugsweise entspricht diese Spiegelachse 43 der Lage der Trennlinie an welcher in einer nachgelagerten Verfahrensstufe die Inkontinenzwindeln vereinzelt werden (siehe Figur 9).

Vor oder nach dem Ausbilden der Öffnungen 53 werden im beispielhaft dargestellten Fall Verschlussmittel 33 in der Fertigungsrichtung zwischen zwei aufeinanderfolgende Öffnungen 53 appliziert. Es handelt sich hierbei um an sich bekannte klebend oder mechanisch haftende Verschlussmittel 33, beispielsweise in Form von streifenförmigen Verschlusstapes. Angedeutet sind mit Bezugszeichen 36 spätere Falzlinien, welche beidseits einer Mittellängsachse 54 der Materialbahn angeordnet sind.

Figur 3 zeigt die Materialbahn 50 nach dem die Materialbahn an einer nicht dargestellten Falzstation um vier in Maschinenrichtung 40 verlaufende Falzlinien 36 zu einer

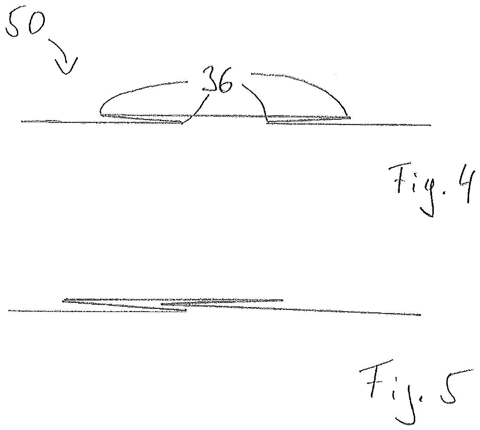

Faltanordnung gefaltet ist, wobei alle vier Falzlinien 36 die ersten Trennlinien 71 kreuzen. Erkennbar ist, dass die Faltanordnung sich nicht über die Materialbahnränder hinauserstreckt. Figur 4 zeigt einen Querschnitt durch die gefaltete Materialbahn 50 der Figur 3 entlang A-A. Erkennbar ist, dass die Faltanordnung in Form einer Omega-Faltung vorliegt. Mithin umfasst die Faltanordnung zwei spiegelbildlich angeordnete Z-Falten. Durch das Einfalten der Materialbahn 50 wird die Breite der geförderten Materialbahn von B1 = 34 cm auf B2 = 17 cm reduziert, also um den Faktor 0,5 reduziert.

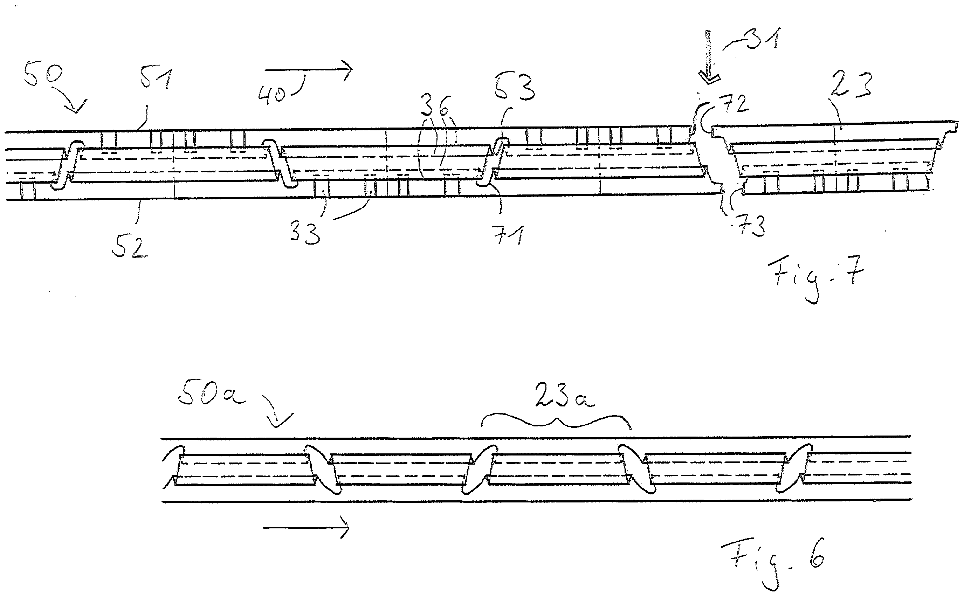

Figur 5 zeigt eine weitere Variante einer vorteilhaften Faltanordnung. Die Faltanordnung umfasst nach dieser Variante zwei Z-Falten, wobei die eine Z-Falte in die andere Z-Falte eingeschoben ist, also Teilabschnitte jeweiliger Z-Falten aufeinander zu liegen kommen. Während Figuren 2 und 3 eine Materialbahn 50 zur Erzeugung von ausschließlich hinteren Windelseitenteilen 20 zeigen, ist in Figur 6 eine Materialbahn 50a dargestellt, welche zur Erzeugung von ausschließlich vorderen Windelseitenteilen 22 bestimmt und geeignet ist. Vorzugsweise weist die Materialbahn 50a im Vergleich zur Materialbahn 50 eine geringere Breite auf. Insbesondere kann die Materialbahn 50a ein von der Materialbahn 50 verschiedenes Material aufweisen. Nach einer weiteren Variante kann die Taktung der (also der Abstand von) in der Maschinenrichtung 40 unmittelbar aufeinander folgenden Materialaussparungen 53 von der der Materialbahn 50 verschieden sein. Das bedeutet, dass die Längserstreckung der von der Materialbahn 50a abzutrennenden Materialbahnabschnitte 23a insbesondere geringer ist als die Längserstreckung der von der Materialbahn 50 abzutrennenden Materialabschnitte 23. Solchenfalls würden die vorderen Windelseitenteile 22 der Windel 2 eine geringere Längserstreckung aufweisen als die der hinteren Windelseitenteile 20.

In einer nur schematisch angedeuteten Vereinzelungsstation 31 wird die in der Maschinenrichtung 40 immer noch endlose Materialbahnen 50 in Materialabschnitte 23 getrennt (Figur 7). In der dargestellten Variante erfolgt dies durch zweite und dritte Trennlinien 72, 73, welche durch Schnitte gebildet werden, die ausgehend von einem jeweiligen Materialbahnrand 51, 52 sich bis zu der ersten Trennlinie 71 in der dargestellten Variante also bis in die Öffnung 53 erstrecken. Die Abtrennung kann an sich durch beliebige Trennverfahren insbesondere Schneiden, Stanzen oder Abreißen erfolgen. Auch könnten nach einer weiteren Variante Schwächungslinien eingebracht werden, die die endgültige Abtrennung der Materialbahnabschnitte durch Ausübung von Zug- oder Druckkräften ermöglichen.

Die Abtrennung der Materialbahnabschnitte 23a von der Materialbahn 50a, mithin die Abtrennung von doppelnutzigen Materialbahnabschnitten 23a für die Bildung von vorderen Windelseitenteilen 20 erfolgt vorzugsweise analog wie zuvor für Materialbahn 50 beschrieben.

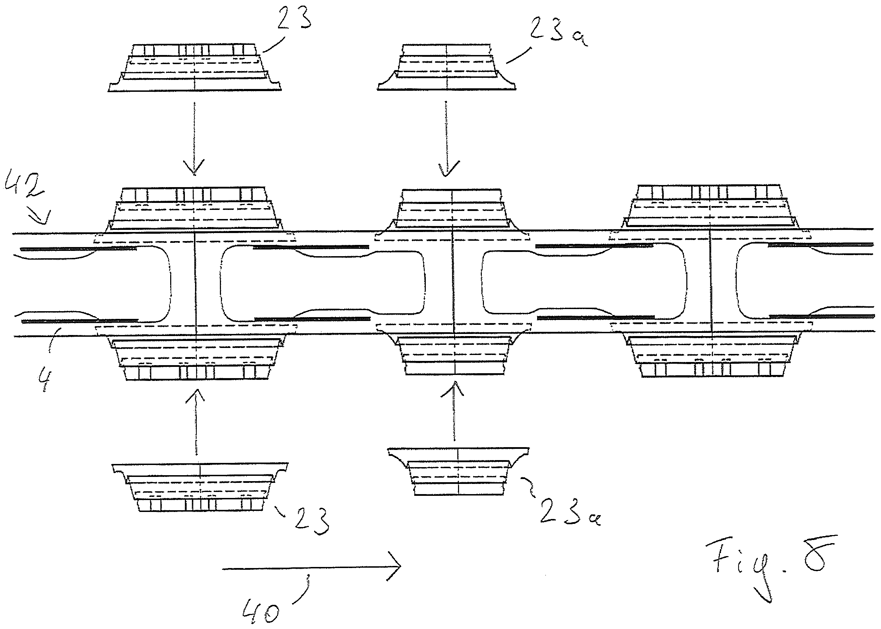

Diese Materialabschnitte 23, 23a können dann mittels geeigneter Applikatoren (nicht dargestellt) auf eine Hauptteilbahn 42, welche entlang der Maschinenrichtung 40 gefördert wird appliziert und dort an Längsrandabschnitten des späteren Windelhauptteils 4 unlösbar befestigt werden (Figur 8).

Schließlich erfolgt an einer nur schematisch angedeuteten Windelvereinzelungsstation 41 die Vereinzelung der zuvor noch endlosen Hauptteilbahn 42 mit daran angefügten doppelnutzigen Materialabschnitten 23, 23a quer zur Maschinenrichtung, wobei die doppelnutzigen Materialbahnabschnitte 23, 23a erst gleichzeitig mit dem Vereinzelungsschnitt in Windelseitenteile 20,22 einer jeweils vereinzelten Windel 2 getrennt werden (Figur 9).

## Patentansprüche

1. Verfahren zum Herstellen von Inkontinenzwegwerfwindeln (2), mit einem Hauptteil (4), bestehend aus einem Vorderbereich (6), einem Rückenbereich (8) und einem in Längsrichtung (28) dazwischen liegenden zwischen den Beinen eines Benutzers zu liegen kommenden Schrittbereich (10), wobei der Hauptteil (4) einen Saugkörper (12) umfasst, und mit beidseits an den Rückenbereich (8) und/oder beidseits an den Vorderbereich (6) angefügten Windelseitenteilen (20, 22), wobei die Windelseitenteile (20, 22) zumindest auf der dem Schrittbereich (10) zugewandten Seite schräg zur Längsrichtung (28) der Inkontinenzwegwerfwindel (2) verlaufende oder konturierte Beinöffnungsbereiche (7) bilden, umfassend die folgenden Schritte
- Fördern einer Windelseitenteile (20, 22) bildenden Materialbahn (50, 50a) in einer Maschinenrichtung (40), wobei die Materialbahn (50, 50a) einen ersten (51) und zweiten Materialbahnrand (52) aufweist
- Ausführen von ersten Schnitten durch die Materialbahn hindurch und schräg zu der Maschinenrichtung (40) zur Bildung von der Materiabahn abzutrennender doppelnutziger Materialbahnabschnitte (23, 23a), welche entlang der Materialbahn (50, 50a) in der Maschinenrichtung (40) alternierend angeordnet sind, derart, dass in der Maschinenrichtung (40) direkt aufeinanderfolgende Materialbahnabschnitte (23, 23a) zueinander in der Ebene um 180° gedreht angeordnet sind, wobei erste Trennlinien (71) der ersten Schnitte sich nicht bis zu den ersten und zweiten Materialbahnrändern (51, 52) der Materialbahn (50a, 50b) erstrecken, so dass die Materialbahn (50, 50a) zunächst endlos bleibt,
- Faltung der Materialbahn (50, 50a) wenigstens um zwei in Maschinenrichtung (40) verlaufende Falzlinien (36) zu einer Faltanordnung, wobei zumindest eine der Falzlinien (36) die ersten Trennlinien (71) kreuzt
- Abtrennen der doppelnutzigen Materialbahnabschnitte (23, 23a) von der Materialbahn (50, 50a) quer oder schräg zu der Maschinenrichtung (40), derart, dass die ersten Trennlinien (71) zumindest bereichsweise die schräg zur Längsrichtung (28) der Inkontinenzwegwerfwindel (2) verlaufenden oder konturierten Beinöffnungsbereiche der Inkontinenzwegwerfwindel bilden können.

2. Verfahren nach Anspruch 1, **dadurch gekennzeichnet, dass** die Faltanordnung eine Z-Falte umfasst.

3. Verfahren nach Anspruch 1 oder 2, **dadurch gekennzeichnet, dass** die Materialbahn (50, 50a) um wenigstens vier in Maschinenrichtung (40) verlaufenden Falzlinien (36) gefaltet ist.

4. Verfahren nach Anspruch 3, **dadurch gekennzeichnet, dass** die in Maschinenrichtung (40) verlaufenden Falzlinien (36) beidseits und insbesondere symmetrisch zu einer Mittellängsachse (54) der Materialbahn (50, 50a) angeordnet sind.

5. Verfahren nach Anspruch 4, **dadurch gekennzeichnet, dass** die Faltanordnung in Form einer Omega-Faltung ausgebildet ist.

6. Verfahren nach einem der vorgenannten Ansprüche, **dadurch gekennzeichnet, dass** bei der Abtrennung der doppelnutzigen Materialbahnabschnitte (23, 23a) von der Materialbahn (50, 50a) die Abtrennung ausgehend von einem jeweiligen Materialbahnrand (51, 52) der Materialbahn (50, 50a) bis zu der ersten Trennlinie (71) erfolgt.

7. Verfahren nach einem der vorgenannten Ansprüche, **dadurch gekennzeichnet, dass** die doppelnutzigen Materialbahnabschnitte (23, 23a) nach der Abtrennung von der Materialbahn (50, 50a) um wenigstens je eine weitere in Maschinenrichtung (40) verlaufende Falzlinie gefaltet werden.

8. Verfahren nach einem der vorstehenden Ansprüche, **dadurch gekennzeichnet, dass** die doppelnutzigen Materialbahnabschnitte (23, 23a) einer in der Maschinenrichtung (40) geförderten Hauptteilbahn (42) zugeführt werden und die Materialbahnabschnitte (23, 23a) zur Bildung von Windelseitenteilen (20, 22) an der Hauptteilbahn (42) angefügt werden.

9. Verfahren nach Anspruch 8, **dadurch gekennzeichnet, dass** die Materialbahn (50, 50a) in der Maschinenrichtung (40) mit einer ersten Geschwindigkeit v1 gefördert wird und die Hauptteilbahn (42) in der Maschinenrichtung (40) mit einer zweiten Geschwindigkeit v2 gefördert wird, wobei gilt v1<v2.

10. Verfahren nach Anspruch 9, **dadurch gekennzeichnet, dass** die doppelnutzigen Materialbahnabschnitte (23, 23a) nach der Abtrennung von der Materialbahn (50, 50a) beschleunigt werden, insbesondere auf eine Geschwindigkeit v3 beschleunigt werden, die im Wesentlichen v2 entspricht.

11. Verfahren nach einem der vorgenannten Ansprüche, **dadurch gekennzeichnet, dass** vor dem Abtrennen der Materialbahnabschnitte (23) von der Materialbahn (50) Verschlussmittel (33) auf die Materialbahn (50) aufgebracht und dort festgelegt werden.

12. Verfahren nach einem der vorgenannten Ansprüche, **dadurch gekennzeichnet, dass** ein doppelnutziger Materialbahnabschnitt (23, 23a) zwei hintere Windelseitenteile oder zwei vordere Windelseitenteile oder ein hinteres und ein vorderes Windelseitenteil bildet.

13. Verfahren nach einem oder mehreren der vorstehenden Ansprüche, **dadurch gekennzeichnet**, wobei die ersten Trennlinien (71) der ersten Schnitte Materialaussparungen bilden, derart dass langestreckte Öffnungen (53) insbesondere in ovaler Form ausgebildet werden.

14. Verfahren nach einem oder mehreren der vorstehenden Ansprüche, **dadurch gekennzeichnet, dass** die Inkontinenzwegwerfwindeln (2) derart gefertigt werden, dass bei in der Maschinenrichtung (40) aufeinanderfolgend geförderten Inkontinenzwegwerfwindeln der Rückenbereich (8) der einen Inkontinenzwegwerfwindel an den Rückenbereich (8) der anderen Inkontinenzwegwerfwindel anschließt und der Vorderbereich (6) der einen Inkontinenzwegwerfwindel an den Vorderbereich (6) der anderen Inkontinenzwegwerfwindel anschließt.

15. Verfahren nach einem oder mehreren der vorstehenden Ansprüche 1-13, **dadurch gekennzeichnet, dass** die Inkontinenzwegwerfwindeln derart gefertigt werden, dass bei in der Maschinenrichtung (40) aufeinanderfolgend geförderten Inkontinenzwegwerfwindeln der Rückenbereich (8) der einen Inkontinenzwegwerfwindel an den Vorderbereich (6) der anderen Inkontinenzwegwerfwindel anschließt.

16. Verfahren nach einem oder mehreren der vorstehenden Ansprüche, **dadurch gekennzeichnet, dass** die Windelseitenteile (20, 22) von Vorderbereich (6) und Rückenbereich (8) verschieden ausgebildet sind.

17. Verfahren nach Anspruch 16, **dadurch gekennzeichnet, dass** die Windelseitenteile (20, 22) von Vorderbereich (6) und Rückenbereich (8) sich hinsichtlich des Materials und/oder des Flächengewichts und/oder der Atmungsaktivität der verwendeten Materialbahn (50, 50a) unterscheiden.

## Claims

1. Method of producing disposable incontinence pads (2) having a main part (4) comprising a front region (6), a rear region (8) and a crotch region (10), which is located between the front and rear regions in the longitudinal direction (28) and ends up located between a user's legs, wherein the main part (4) comprises an absorbent body (12), and having pad side parts (20, 22) joined to the rear region (8) on either side and/or joined to the front region (6) on either side, wherein, at least on the side directed towards the crotch region (10), the pad side parts (20, 22) form leg-opening regions (7) which are contoured or run obliquely in relation to the longitudinal direction (28) of the disposable incontinence pad (2), the method comprising the following steps
- conveying in a machine direction (40) a material web (50, 50a) which forms pad side parts (20, 22), wherein the material web (50, 50a) has a first material-web periphery (51) and second material-web periphery (52),
- making first cuts through the material web, and obliquely in relation to the machine direction (40), to form double-use material-web portions (23, 23a) which can be separated off from the material web and alternate along the material web (50, 50a) in the machine direction (40) such that material web portions (23, 23a) which follow directly one after the other in the machine direction (40) are arranged in a state in which they have been rotated through 180° in the plane in relation to one another, wherein first separating lines (71) of the first cuts do not extend as far as the first and second peripheries (51, 52) of the material web (50a, 50b), and therefore the material web (50, 50a) in the first instance remains endless,
- folding the material web (50, 50a) at least around two folding lines (36), which run in the machine direction (40), to give a folded arrangement, wherein at least one of the folding lines (36) crosses over the first separating lines (71),
- separating off the double-use material-web portions (23, 23a) from the material web (50, 50a) transversely or obliquely in relation to the machine direction (40) such that the first separating lines (71), at least in certain regions, can form the leg-opening regions of the disposable incontinence pad, said leg-opening regions being contoured or running obliquely in relation to the longitudinal direction (28) of the disposable incontinence pad (2).

2. Method according to Claim 1, **characterized in that** the folded arrangement comprises a Z-shaped fold.

3. Method according to Claim 1 or 2, **characterized in that** the material web (50, 50a) is folded around at least four folding lines (36), which run in the machine direction (40).

4. Method according to Claim 3, **characterized in that** the folding lines (36), which run in the machine direction (40), are arranged on either side and in particular symmetrically in relation to a centre longitudinal axis (54) of the material web (50, 50a).

5. Method according to Claim 4, **characterized in that** the folded arrangement is in the form of an omega-shaped folded formation.

6. Method according to one of the preceding claims, **characterized in that**, when the double-use material-web portions (23, 23a) are separated off from the material web (50, 50a), the separating-off operation starts from a respective periphery (51, 52) of the material web (50, 50a) and extends as far as the first separating line (71).

7. Method according to one of the preceding claims, **characterized in that**, once they have been separated off from the material web (50, 50a), the double-use material-web portions (23, 23a) are folded around at least in each case one further folding line running in the machine direction (40).

8. Method according to one of the preceding claims, **characterized in that** the double-use material-web portions (23, 23a) are fed to a main-part web (42) conveyed in the machine direction (40) and the material-web portions (23, 23a) are joined to the main-part web (42) to form pad side parts (20, 22).

9. Method according to Claim 8, **characterized in that** the material web (50, 50a) is conveyed in the machine direction (40) at a first speed v1 and the main-part web (42) is conveyed in the machine direction (40) at a second speed v2, where v1 < v2.

10. Method according to Claim 9, **characterized in that**, once they have been separated off from the material web (50, 50a), the double-use material-web portions (23, 23a) are accelerated, in particular are accelerated to a speed v3, which corresponds essentially to v2.

11. Method according to one of the preceding claims, **characterized in that**, prior to the material-web portions (23) being separated off from the material web (50), closure means (33) are applied to the material web (50) and secured there.

12. Method according to one of the preceding claims, **characterized in that** a double-use material-web portion (23, 23a) forms two rear pad side parts or two front pad side parts or one rear, and one front, pad side part.

13. Method according to one or more of the preceding claims, **characterized in** wherein the first separating lines (71) of the first cuts form material cutouts such that in particular oval elongate openings (53) are formed.

14. Method according to one or more of the preceding claims, **characterized in that** the disposable incontinence pads (2) are produced such that, in the case of disposable incontinence pads being conveyed one after the other in the machine direction (40), the rear region (8) of the one disposable incontinence pad adjoins the rear region (8) of the other disposable incontinence pad and the front region (6) of the one disposable incontinence pad adjoins the front region (6) of the other disposable incontinence pad.

15. Method according to one or more of preceding Claims 1-13, **characterized in that** the disposable incontinence pads are produced such that, in the case of disposable incontinence pads being conveyed one after the other in the machine direction (40), the rear region (8) of the one disposable incontinence pad adjoins the front region (6) of the other disposable incontinence pad.

16. Method according to one or more of the preceding claims, **characterized in that** the pad side parts (20, 22) of the front region (6) and rear region (8) are designed differently.

17. Method according to Claim 16, **characterized in that** the pad side parts (20, 22) of the front region (6) and rear region (8) differ in respect of the material and/or of the weight per unit area and/or of the breathability of the material web (50, 50a) used.

## Revendications

1. Procédé de fabrication de couches jetables pour incontinence (2), avec une partie principale (4) se composant d'une région antérieure (6), d'une région dorsale (8) et d'une région d'entrejambe (10) située entre celles-ci en direction longitudinale et venant se placer entre les jambes d'un utilisateur, dans lequel la partie principale (4) comprend un corps aspirant (12), et avec des parties latérales de couche (20, 22) jointes de part et d'autre à la région dorsale (8) et/ou de part et d'autre à la région antérieure (6), dans lequel les parties latérales de couche (20, 22) forment au moins sur le côté tourné vers la région d'entrejambe des régions d'ouverture de jambe (7) orientées en oblique par rapport à la direction longitudinale (28) de la couche jetable pour incontinence (2) ou coupées en forme, comprenant les opérations suivantes:
- transporter une bande de matière (50, 50a) formant des parties latérales de couche (20, 22) dans une direction machine (40), dans lequel la bande de matière (50, 50a) présente un premier (51) et un second (52) bords de bande de matière,
- effectuer des premières coupes à travers la bande de matière et en oblique par rapport à la direction machine (40) pour la formation de parties de bande de matière à double usage (23, 23a) à détacher de la bande de matière, qui sont disposées en alternance le long de la bande de matière (50, 50a) dans la direction machine (40), de telle manière que des parties de bande de matière (23, 23a) se succédant directement dans la direction machine (40) soient disposées de façon tournée de 180° l'une par rapport à l'autre dans le plan, dans lequel des premières lignes de séparation (71) des premières coupes ne s'étendent pas jusqu'aux premiers et seconds bords de bande de matière (51, 52) de la bande de matière (50, 50a), de telle manière que la bande de matière (50, 50a) reste d'abord sans fin,
- plier la bande de matière (50, 50a) au moins autour de deux lignes de pliage (36) s'étendant dans la direction machine (40) en un agencement de plis, dans lequel au moins une des lignes de pliage (36) croise les premières lignes de séparation (71),
- séparer les parties de bande de matière à double usage (23, 23a) de la bande de matière (50, 50a) transversalement ou en oblique par rapport à la direction machine (40), de telle manière que les premières lignes de séparation (71) puissent former au moins localement les régions d'ouverture de jambe de la couche jetable pour incontinence s'étendant en oblique par rapport à la direction longitudinale (28) de la couche jetable pour incontinence (2) ou coupées en forme.

2. Procédé selon la revendication 1, **caractérisé en ce que** l'agencement de plis comprend un pli en Z.

3. Procédé selon la revendication 1 ou 2, **caractérisé en ce que** la bande de matière (50, 50a) est pliée autour d'au moins quatre lignes de pliage (36) s'étendant dans la direction machine (40).

4. Procédé selon la revendication 3, **caractérisé en ce que** les lignes de pliage (36) s'étendant dans la direction machine (40) sont disposées de part et d'autre et en particulier de façon symétrique par rapport à un axe longitudinal central (54) de la bande de matière (50, 50a).

5. Procédé selon la revendication 4, **caractérisé en ce que** l'agencement de plis est réalisé en forme de pliage oméga.

6. Procédé selon l'une quelconque des revendications précédentes, **caractérisé en ce que** lors de la séparation des parties de bande de matière à double usage (23, 23a) de la bande de matière (50, 50a), on effectue la séparation à partir du bord de bande de matière respectif (51, 52) de la bande de matière (50, 50a) jusqu'à la première ligne de séparation (71).

7. Procédé selon l'une quelconque des revendications précédentes, **caractérisé en ce que** l'on plie les parties de bande de matière à double usage (23, 23a) après la séparation de la bande de matière (50, 50a) autour d'au moins chaque fois une autre ligne de pliage s'étendant dans la direction machine (40).

8. Procédé selon l'une quelconque des revendications précédentes, **caractérisé en ce que** l'on amène les parties de bande de matière à double usage (23, 23a) à une bande de partie principale (42) transportée dans la direction machine (40) et on assemble les parties de bande de matière (23, 23a) à la bande de partie principale (42) pour la formation des parties latérales de couche (20, 22).

9. Procédé selon la revendication 8, **caractérisé en ce que** l'on transporte la bande de matière (50, 50a) dans la direction machine (40) avec une première vitesse v1 et on transporte la bande de partie principale (42) dans la direction machine (40) avec une seconde vitesse v2, pour lesquelles on a v1 < v2.

10. Procédé selon la revendication 9, **caractérisé en ce que** l'on accélère les parties de bande de matière à double usage (23, 23a) après la séparation de la bande de matière (50, 50a), en particulier on les accélère à une vitesse v3, qui correspond essentiellement à v2.

11. Procédé selon l'une quelconque des revendications précédentes, **caractérisé en ce que** l'on applique et on fixe des moyens de fermeture ((33) sur la bande de matière (50) avant la séparation des parties de bande de matière (23) de la bande de matière (50).

12. Procédé selon l'une quelconque des revendications précédentes, **caractérisé en ce qu'**une partie de bande de matière à double usage (23, 23a) forme deux parties latérales de couche postérieures ou deux parties latérales de couche antérieures ou une partie latérale de couche postérieure et une antérieure.

13. Procédé selon une ou plusieurs des revendications précédentes, **caractérisé en ce que** les premières lignes de séparation (71) des premières coupes forment des découpes de matière, de telle manière que des ouvertures allongées (53) soient formées, en particulier sous forme ovale.

14. Procédé selon une ou plusieurs des revendications précédentes, caractérisé en ce dans lequel l'on fabrique les couches jetables pour incontinence (2) de telle manière que, dans des couches jetables pour incontinence transportées l'une à la suite de l'autre dans la direction machine (40), la région dorsale (8) d'une couche jetable pour incontinence se raccorde à la région dorsale (8) de l'autre couche jetable pour incontinence et que la région antérieure (6) d'une couche jetable pour incontinence se raccorde à la région antérieure (6) de l'autre couche jetable pour incontinence.

15. Procédé selon une ou plusieurs des revendications précédentes 1 à 13, **caractérisé en ce que** l'on fabrique les couches jetables pour incontinence de telle manière que, dans des couches jetables pour incontinence transportées l'une à la suite de l'autre dans la direction machine (40), la région dorsale (8) d'une couche jetable pour incontinence se raccorde à la région antérieure (6) de l'autre couche jetable pour incontinence.

16. Procédé selon une ou plusieurs des revendications précédentes, **caractérisé en ce que** les parties latérales de couche (20, 22) de la région antérieure (6) et de la région dorsale (8) sont formées de façon différente.

17. Procédé selon la revendication 16, **caractérisé en ce que** les parties latérales de couche (20, 22) de la région antérieure (6) et de la région dorsale (8) se différencient en ce qui concerne la matière et/ou le poids par unité de surface et/ou l'activité de respiration de la bande de matière utilisée (50, 50a).
